# EUROPEAN PATENT APPLICATION

(11) **EP 4 718 369 A1**
(43) Date of publication of application: **01.04.2026**
(21) Application number: 25199024.8
(22) Date of filing: 29.08.2025
(51) Int. Cl.: G06T 3/4053, G06V 10/82

(54) **IMAGE PROCESSING METHOD AND ELECTRONIC DEVICE**

(30) Priority: 27.09.2024 TW 113137173
(71) Applicant: ASUSTek Computer Inc., Peitou, Taipei-City 112 (TW)
(72) Inventor: CHEN, Yu-Cheng, 112 Taipei City (TW)
(74) Representative: Viering, Jentschura & Partner mbB Patent- und Rechtsanwälte

(57) **Abstract**

An image processing method and an electronic device (10) are provided. The electronic device (10) performs the image processing method. The image processing method includes: inputting a low-resolution image into a super-resolution model (121); using the super-resolution model (121) to perform an interpolation process on the low-resolution image to generate a first intermediate image; using the super-resolution model (121) to perform a convolution activation process on the low-resolution image to generate a second intermediate image; and performing an addition operation on the first intermediate image and the second intermediate image to generate a high-resolution image.

## Description

### BACKGROUND OF THE INVENTION

### Field of the Invention

The disclosure relates to an image processing method and an electronic device, applied to endoscopic images based on deep learning-based super resolution imaging (super resolution imaging).

### Description of the Related Art

Endoscopic inspection instrument is a technology developed mainly in response to internal examination of human bodies. The endoscopic inspection instrument enters a human body through various channels and observes the internal status of the human body. Endoscopic images captured by the endoscopic inspection instrument is provided to physicians for reference, to determine whether there is any lesion. In terms of the endoscopic images, in comparison with a low-resolution image, a high-resolution image includes more detailed information structures, and therefore, the high-resolution image can provide more information to a physician for reference. However, in existing technologies, an image capture apparatus, for example, an image capture apparatus including Olympus EVIS X1, or CV1500, is needed to obtain a high-resolution image. However, these apparatuses have the disadvantage of being expensive.

In addition, to improve the resolution of an endoscopic image, an interpolation method, for example, a linear interpolation (linear interpolation) method or a bicubic interpolation (bicubic interpolation) method is used to improve the resolution of the endoscopic image in a conventional technology. However, the interpolation method results in blurred images, which cannot provide an effective reference.

### BRIEF SUMMARY OF THE INVENTION

The disclosure provides an image processing method, including: inputting a low-resolution image into a super-resolution model; using the super-resolution model to perform an interpolation process on the low-resolution image to generate a first intermediate image; using the super-resolution model to perform a convolution activation process on the low-resolution image to generate a second intermediate image; and performing an addition operation on the first intermediate image and the second intermediate image to generate a high-resolution image.

The disclosure further provides an electronic device, including a storage device and a processing device. The storage device stores a low-resolution image. The processing device is electrically connected to the storage device and incorporates a super-resolution model. The processing device inputs the low-resolution image into the super-resolution model, uses the super-resolution model to separately perform an interpolation process and a convolution activation process on the low-resolution image to separately generate a first intermediate image and a second intermediate image, and performs an addition operation on the first intermediate image and the second intermediate image to generate a high-resolution image.

In conclusion, a high-resolution image, which is only obtainable with high-level hardware, can be obtained by using the image processing method and the electronic device in the disclosure through computing with artificial intelligence (AI) software, without using expensive hardware. In addition, the generated high-resolution image has sharp edges and are blur-free. Therefore, when the disclosure is applied to endoscopic images, a 4K to 8K high-resolution image can be obtained by an apparatus with a 1080P output source.

### BRIEF DESCRIPTION OF THE DRAWINGS

FIG. 1 is a schematic block diagram of an electronic device according to an embodiment of the disclosure;
FIG. 2 is a schematic flowchart of an image processing method according to an embodiment of the disclosure; and
FIG. 3 is a schematic block diagram of an electronic device electrically connected to an inspection instrument according to an embodiment of the disclosure.

### DETAILED DESCRIPTION OF THE EMBODIMENTS

The embodiments of the disclosure are described with reference to relevant drawings. In addition, some elements or structures are omitted in the drawings in the embodiments, to clearly show technical features of the disclosure. In these drawings, the same numerals indicate the same or similar elements or circuits. It is to be noted that terms such as "first" and "second" are used to describe various elements, components, regions, or functions herein, but the elements, components, regions, and/or functions are not limited to these terms. These terms are merely used to distinguish one element, component, region, or function from another element, component, region, or function.

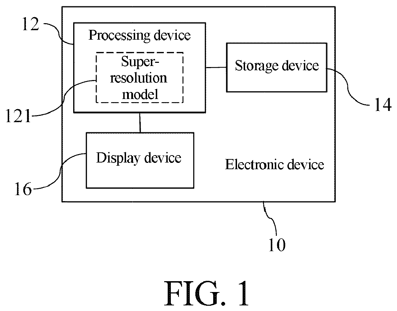

Refer to FIG. 1. An electronic device 10 includes a processing device 12 and a storage device 14. The storage device 14 stores one low-resolution image or a plurality of low-resolution images. The processing device 12 is electrically connected to the storage device 14 and incorporates a super-resolution model (super-resolution model) 121. The processing device 12 reads the low-resolution image from the storage device 14, and inputs the low-resolution image into the super-resolution model 121 for image processing. The processing device 12 uses the super-resolution model 121 to separately perform an interpolation process and a convolution activation process on the low-resolution image to separately generate a first intermediate image and a second intermediate image, and performs an addition operation on the first intermediate image and the second intermediate image to generate a high-resolution image.

In an embodiment, with reference to FIG. 1, the electronic device 10 further includes a display device 16. The display device 16 is electrically connected to the processing device 12. The processing device 12 may further provides a user interface (not shown in the figure) displayed on the display device 16, to perform the entire process of the image processing through the user interface. After the super-resolution model 121 generates the high-resolution image, the processing device 12 may further directly present the high-resolution image on the user interface of the display device 16 for viewing by a user.

In an embodiment, the electronic device 10 is an electronic apparatus (a computer apparatus) that can operate independently, for example, a personal computer, a notebook computer, or a tablet computer, but the disclosure is not limited thereto.

In an embodiment, the processing device 12 is a central processing unit (central processing unit, CPU), an embedded controller (embedded controller, EC), another general-purpose or special-purpose microprocessor (microprocessor), a microcontroller (microcontroller), a micro control unit (micro control unit, MCU), a digital signal processor (digital signal processor, DSP), a programmable controller, an application specific integrated circuit (application specific integrated circuit, ASIC), another similar device, or a combination of the above devices, and the disclosure is not limited thereto.

In an embodiment, the storage device 14 may be a fixed or movable random access memory (random access memory, RAM), read-only memory (read-only memory, ROM), flash memory (flash memory), hard disk drive (hard disk drive, HDD), solid state drive (solid state drive, SSD) in any form, another similar device, or a combination of the above devices, to be configured to store any image, picture, data, or the like that is needed by the processing device 12, but the disclosure is not limited thereto.

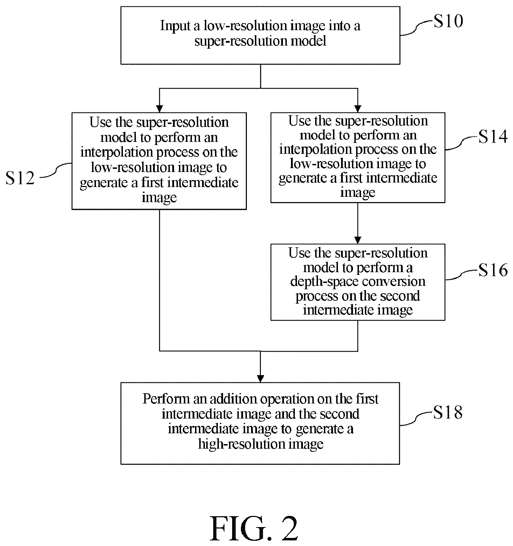

In the electronic device 10, the processing device 12 performs an image processing method by using software. Refer to both FIG. 1 and FIG. 2. As shown in step S10, the processing device 12 inputs a low-resolution image into the super-resolution model 121. Descriptions are provided herein by using an example in which a matrix size of the low-resolution image is [1000*1000*3], but the disclosure is not limited thereto. As shown in step S12, the processing device 12 uses the super-resolution model 121 to perform an interpolation process on the low-resolution image to generate a first intermediate image. In an embodiment, the interpolation process is to process the low-resolution image by using a bilinear interpolation (bilinear interpolation) method, to upscale the image to generate the first intermediate image. In this case, a matrix size of the first intermediate image obtained through the interpolation process is [4000*4000*3]. As shown in step S14, the processing device 12 further uses the super-resolution model 121 to perform a convolution activation process on the same low-resolution image to generate a second intermediate image. In an embodiment, the convolution activation process includes processing by 16 convolutional layers and activation function layers, where the activation function layer is a parametric rectified linear unit (parametric rectified linear unit, PReLU), to perform detailed processing on the low-resolution image to generate the second intermediate image. In this case, a matrix size of the second intermediate image obtained through the convolution activation process is [1000*1000*48]. As shown in step S16, after the convolution activation process is performed on the low-resolution image, the super-resolution model 121 is used to perform a depth-space conversion process on the second intermediate image, so that the second intermediate image that is generated through the processing and the first intermediate image have a same image size. In this case, the matrix size of the second intermediate image obtained through the depth-space conversion process is converted to [4000*4000*3], and this image size is the same as that of the first intermediate image. Finally, as shown in step S18, the processing device 12 uses the super-resolution model 121 to perform an addition operation on the first intermediate image and the second intermediate image to generate a high-resolution image. In this case, a matrix size of the high-resolution image is [4000*4000*3]. Therefore, in the disclosure, after the foregoing image processing, a 1000*1000 RGB color image (low-resolution image) is upscaled by four times to obtain a high-resolution image.

In an embodiment, the low-resolution image is a low-resolution endoscopic image, and the high-resolution image is a high-resolution endoscopic image.

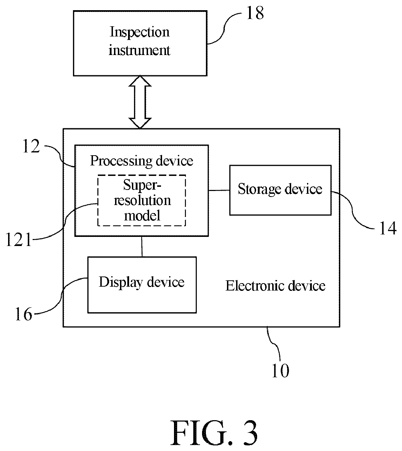

In an embodiment, with reference to FIG. 3, the electronic device 12 is further electrically connected to an inspection instrument 18. For example, the processing device 12 is connected to the inspection instrument 18 through a high definition multimedia interface (HDMI) or a serial digital interface (SDI). The inspection instrument 18 examines a target and generates a real-time image. In this case, the real-time image is used as the low-resolution image. The inspection instrument 18 is an endoscopic system, for example, a colonoscopy inspection instrument. In this case, the target is intestine. The low-resolution image is a low-resolution endoscopic image, and the high-resolution image is a high-resolution endoscopic image. Moreover, in addition to being directly used as the low-resolution image, the real-time image generated when the inspection instrument 18 examines the target may alternatively be stored in the storage device 14 first to be used as a to-be-processed low-resolution image. Then, the processing device 12 reads the low-resolution image from the storage device 14 and performs subsequent image processing.

Refer to FIG. 1. Before using the super-resolution model 121 to perform image processing, the processing device 12 first performs a step of model training. During the training, both the high-resolution image and the low-resolution image are used as training data. The resolution of the high-resolution image may be 1080P to 4K, and an image type is not limited. A corresponding image type may be selected depending on an application field of the super-resolution model 121. For example, if the super-resolution model 121 is to be used in an endoscopic system, an endoscopic image may be used as the training data. The low-resolution image is obtained by reducing the resolution of the high-resolution image, adding noise to the high-resolution image, compressing the high-resolution image, or the like using software. Then, the training data is input into the super-resolution model 121 for training. The low-resolution image is used as input, an output result of the super-resolution model 121 is used as output, and the high-resolution image is used as marked training data. On this basis, iterative training is performed, and a trained super-resolution model 121 can be obtained. The trained super-resolution model 121 is used subsequently for image processing, to convert a low-resolution image to a high-resolution image.

In conclusion, a high-resolution image, which is only obtainable with high-level hardware, can be obtained by using the image processing method and the electronic device in the disclosure through computing with artificial intelligence (AI) software, without using expensive hardware. In addition, the generated high-resolution image has sharp edges and are blur-free. Therefore, when the disclosure is applied in an endoscopic image, a 4K to 8K high-resolution image can be obtained by an apparatus with a 1080P output source, to effectively provide a more accurate reference base for a physician. In addition, a computing level of the processing device used in the electronic device can be run smoothly with only a built-in display card of an electronic computer.

## Claims

1. An image processing method, comprising:
inputting a low-resolution image into a super-resolution model (121);
using the super-resolution model (121) to perform an interpolation process on the low-resolution image to generate a first intermediate image;
using the super-resolution model (121) to perform a convolution activation process on the low-resolution image to generate a second intermediate image; and
performing an addition operation on the first intermediate image and the second intermediate image to generate a high-resolution image.

2. The image processing method according to claim 1, wherein the low-resolution image is a real-time image generated by an inspection instrument (18) when examining a target.

3. The image processing method according to claim 2, wherein the inspection instrument (18) is an endoscopic system, the low-resolution image is a low-resolution endoscopic image, and the high-resolution image is a high-resolution endoscopic image.

4. The image processing method according to claim 1, wherein the low-resolution image is a low-resolution endoscopic image, and the high-resolution image is a high-resolution endoscopic image.

5. The image processing method according to claim 1, wherein the interpolation process is to process the low-resolution image by using a bilinear interpolation method to generate the second intermediate image.

6. The image processing method according to claim 1, wherein the convolution activation process further comprises detailed processing by a plurality of convolutional layers and activation function layers to generate the second intermediate image.

7. The image processing method according to claim 1, wherein the first intermediate image and the second intermediate image have a same image size.

8. The image processing method according to claim 7, wherein after the convolution activation process is performed on the low-resolution image by using the super-resolution model (121), a depth-space conversion process is further performed, so that the generated second intermediate image and the first intermediate image have the same image size.

9. An electronic device (10), comprising:
a storage device (14), storing a low-resolution image; and
a processing device (12), electrically connected to the storage device (14) and incorporating a super-resolution model (121), wherein the processing device (12) inputs the low-resolution image into the super-resolution model (121), uses the super-resolution model (121) to separately perform an interpolation process and a convolution activation process on the low-resolution image to separately generate a first intermediate image and a second intermediate image, and performs an addition operation on the first intermediate image and the second intermediate image to generate a high-resolution image.

10. The electronic device (10) according to claim 9, wherein the electronic device (10) is electrically connected to an inspection instrument (18), the inspection instrument (18) examines a target and generates a real-time image, and the real-time image is used as the low-resolution image.

11. The electronic device (10) according to claim 10, wherein the inspection instrument (18) is an endoscopic system, the low-resolution image is a low-resolution endoscopic image, and the high-resolution image is a high-resolution endoscopic image.

12. The electronic device (10) according to claim 9, wherein the low-resolution image is a low-resolution endoscopic image, and the high-resolution image is a high-resolution endoscopic image.

13. The electronic device (10) according to claim 9, wherein the interpolation process is performed on the low-resolution image by using the super-resolution model (121) based on a bilinear interpolation method to generate the second intermediate image.

14. The electronic device (10) according to claim 9, wherein the convolution activation process further comprises detailed processing by a plurality of convolutional layers and activation function layers to generate the second intermediate image.

15. The electronic device (10) according to claim 9, wherein the first intermediate image and the second intermediate image have a same image size.

16. The electronic device (10) according to claim 15, wherein after the convolution activation process is performed on the low-resolution image by using the super-resolution model (121), a depth-space conversion process is further performed, so that the generated second intermediate image and the first intermediate image have the same image size.
